# EUROPEAN PATENT APPLICATION

(11) **EP 4 273 810 A1**
(43) Date of publication of application: **08.11.2023**
(21) Application number: 21912471.6
(22) Date of filing: 20.01.2021
(51) Int. Cl.: G06T 17/00, G06F 16/904, A61B 18/12

(54) **RADIO FREQUENCY OPERATION PROMPTING METHOD, ELECTRONIC DEVICE, AND COMPUTER-READABLE STORAGE MEDIUM**

(30) Priority: 31.12.2020 CN 202011641885
(71) Applicant: Hangzhou Broncus Medical Co., Ltd., Hangzhou, Zhejiang 310051 (CN)
(72) Inventor: XU, Hong, Hangzhou, Zhejiang 310051 (CN)
(74) Representative: Petraz, Gilberto Luigi
(86) International application number: PCT/CN2021/072959
(87) International publication number: WO 2022/141691

(57) **Abstract**

Disclosed are a radio frequency operation prompting method, an electronic device, and a computer-readable storage medium. The method includes acquiring physical characteristic data of an operating position in an object of a radio frequency operation in real time by multiple probes, obtaining a physical characteristic field of the object of the radio frequency operation according to the physical characteristic data acquired in real time; and obtaining a change of range of a to-be-operated area in a target operating area according to an initial range of the target operating area in the object of the radio frequency operation and a change of value of the physical characteristic data in the physical characteristic field, and displaying the change of range through a three-dimensional model. In the present invention, visual prompting of the change of range of the to-be-operated area is achieved, and the accuracy and intelligence of determining the to-be-operated area is increased, thereby improving the effectiveness of information prompts, and thus improving the success rate and effect of the radio frequency operation.

## Description

### TECHNICAL FIELD

Embodiments of the present invention relates to the technical field of data processing, and particularly to a radio frequency operation prompting method, an electronic device and a computer-readable storage medium.

### BACKGROUND

In the radio frequency technology, a radio frequency probe enters an operating position in an object of a radio frequency operation under the guidance of an image, a radio frequency host transmits a radio frequency signal and the radio frequency signal is applied onto the object of the radio frequency operation, to implement the radio frequency operation. During the radio frequency operation, the control of the effect of the radio frequency operation is also an important guarantee for the final good effect of the radio frequency operation.

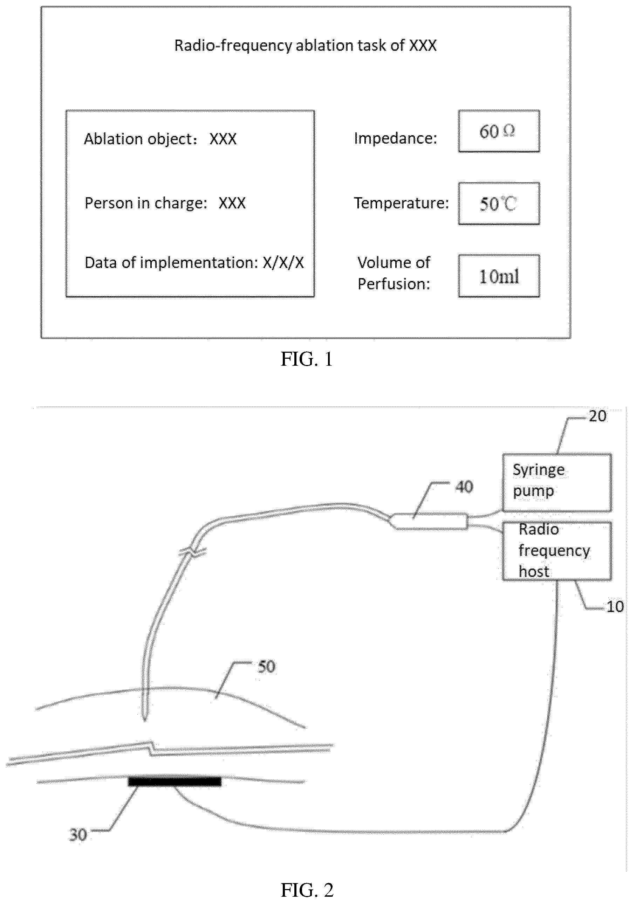

In the prior art, as shown in FIG. 1, when a radio frequency host implements a radio frequency operation, generally, prompt information is displayed in a simple digital form on the display interface, to indicate to a user the current status of the radio frequency operation. However, this way of prompting is not accurate enough, the prompt information is simple and less indicative, and the prompting effect is poor, which in turn affect the effect of the radio frequency operation.

### SUMMARY

### Technical Problem

An embodiment of the present application provides a radio frequency operation prompting method, an electronic device, and a computer-readable storage medium, with which visual prompting of the change of range of a to-be-operated area is realized, thereby improving the effectiveness of information prompts, and thus improving the success rate and effect of the radio frequency operation.

### Technical solution

In an aspect, an embodiment of the present application provides a radio frequency operation prompting method, applicable to a computer terminal. The method includes:
acquiring physical characteristic data of an operating position in an object of a radio frequency operation in real time by multiple probes;
obtaining a physical characteristic field of the object of the radio frequency operation according to the physical characteristic data acquired in real time; and
obtaining a change of range of a to-be-operated area in a target operating area according to an initial range of the target operating area in the object of the radio frequency operation and a change of value of the physical characteristic data in the physical characteristic field, and displaying the change of range through a three-dimensional model.

In an aspect, an embodiment of the present application further provides a radio frequency operation prompting device, which includes:
an acquisition module, configured to acquire physical characteristic data of an operating position in an object of a radio frequency operation in real time by multiple probes;
a processing module, configured to obtain a physical characteristic field of the object of the radio frequency operation according to the physical characteristic data acquired in real time, and
obtain a change of range of a to-be-operated area in a target operating area according to an initial range of the target operating area in the object of the radio frequency operation and a change of value of the physical characteristic data in the physical characteristic field; and
a display module, configured to display the change of range through a three-dimensional model.

In an aspect, an embodiment of the present application further provides an electronic device, which includes a storage and a processor, wherein
the storage stores an executable program code; and
the processor is coupled to the storage, and configured to call the executable program code stored in the storage, and implement the radio frequency operation prompting method provided in the above embodiments.

In an aspect, an embodiment of the present application further provides a non-transitory computer-readable storage medium, on which a computer program is stored, wherein when the computer program is executed by a processor, the radio frequency operation prompting method provided in the above embodiments is implemented.

### Beneficial effects

According to various embodiments provided in the present application, multiple pieces of physical characteristic data of an operating position in an object of a radio frequency operation are acquired in real time by multiple probes, a physical characteristic field of the object of the radio frequency operation is obtained according to these pieces of data, then the change of range of a to-be-operated area in a target operating area is obtained according to an initial range of the target operating area in the object of the radio frequency operation and the change of value of the physical characteristic data in the physical characteristic field, and displayed through a three-dimensional model. As a result, the visual prompting of the change of range of the to-be-operated area is realized, the content of the prompt information is much rich, intuitive and vivid, and the accuracy and intelligence of determining the to-be-operated area is increased, thereby improving the effectiveness of information prompts, and thus improving the success rate and effect of the radio frequency operation.

### BRIEF DESCRIPTION OF THE DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present application. Other drawings can be obtained by persons of ordinary skill in the art based on these drawings without creative efforts.
FIG. 1 is a schematic diagram of a conventional radio frequency operation prompting interface;
FIG. 2 shows an application environment of a radio frequency operation prompting method provided in an embodiment of the present application;
FIG. 3 shows a flow chart of a radio frequency operation prompting method provided in an embodiment of the present application;
FIG. 4 is a schematic view of a tip of a radio frequency operation catheter in a radio frequency operation prompting method provided in an embodiment of the present application;
FIG. 5 shows a flow chart of a radio frequency operation prompting method provided in another embodiment of the present application;
FIG. 6 is a schematic structural diagram of a radio frequency operation prompting device provided in an embodiment of the present application; and
FIG. 7 is a schematic diagram showing a hardware structure of an electronic device provided in an embodiment of the present application.

### DESCRIPTION OF THE EMBODIMENTS

In order to make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present application. All other embodiments obtained by ordinary persons skilled in the art based on the embodiments of the present invention without creative efforts shall fall within the protection scope of the present invention.

Fig. 2 is a schematic view showing an application scenario of a radio frequency operation prompting method provided in an embodiment of the present application. The radio frequency operation prompting method can be implemented by a radio frequency host 10 shown in FIG. 2, or implemented by other computer equipment that has established a data connection with the radio frequency host 10.

As shown in FIG. 2, the radio frequency host 10 is connected to a syringe pump 20, a neutral electrode 30 and a radio frequency operation catheter 40. The radio frequency host 10 is provided with a built-in display screen (not shown).

Specifically, before an operation task is implemented, an energy emitting end of the radio frequency operation catheter 40 for generating and outputting radio frequency energy and an extension tube (not shown) of the syringe pump 20 are inserted into the body of an object 50 (such as an abnormal tissue mass). Then, the neutral electrode 30 is brought into contact with the skin surface of the object 50. A radio frequency current flows through the radio frequency operation catheter 40, the object 50 and the neutral electrode 30, to form a loop.

When the operation task is triggered, the radio frequency operation catheter 40 is controlled by the radio frequency host 10 to output radio frequency energy to an operation site by discharging, so as to implement a radio frequency operation on the operation site. Moreover, the syringe pump 20 performs a perfusion operation on the object through the extension tube, wherein physiological saline is infused into the operation site, to adjust the impedance and temperature of the operation site.

Moreover, the radio frequency host 10 acquires physical characteristic data of an operating position in the object of the radio frequency operation in real time by multiple probes (not shown) provided at a tip of the radio frequency operation catheter 40, obtains a physical characteristic field of the object of the radio frequency operation according to the physical characteristic data acquired in real time; and then obtains the change of range of a to-be-operated area in a target operating area according to an initial range of the target operating area in the object of the radio frequency operation and the change of value of the physical characteristic data in the physical characteristic field, and displays the change of range through a three-dimensional model.

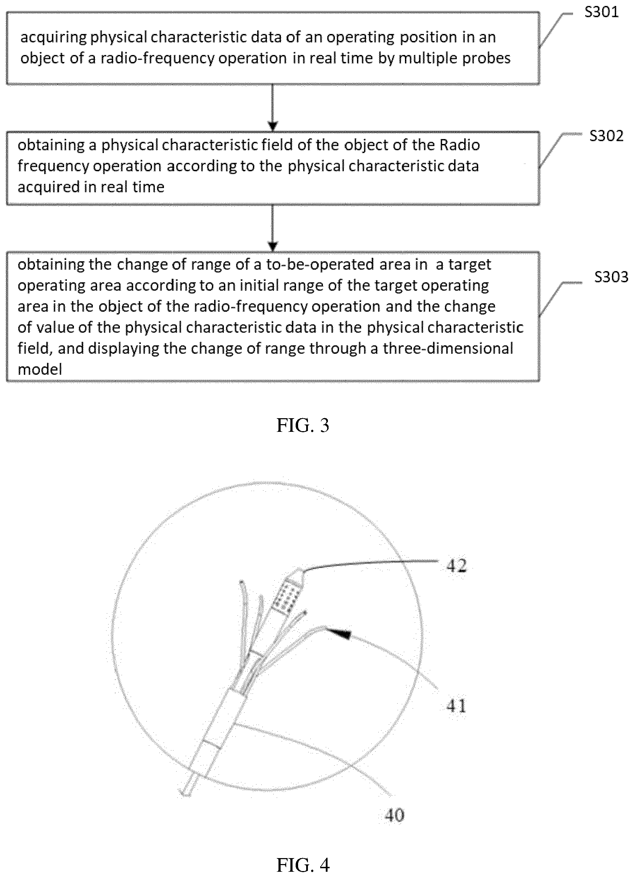

Fig. 3 shows a flow chart of a radio frequency operation prompting method provided in an embodiment of the present application. The method can be implemented by the radio frequency host 10 shown in FIG. 2, or implemented by other computer terminals connected thereto. For ease of description, in the following embodiments, the radio frequency host 10 is used as an implementation body. As shown in FIG. 3, the method specifically includes:

Step S301: acquiring physical characteristic data of an operating position in an object of a radio frequency operation in real time by multiple probes.

As shown in FIG. 4, multiple probes 41 are arranged around a central electrode 42 for outputting radio frequency energy provided at a tip of the radio frequency operation catheter 40, and located on different planes, to form a claw-shaped structure collectively. Each probe is provided with a physical characteristic data acquiring device, configured to acquire physical characteristic data of a pierced or touched position.

Particularly, when the radio frequency operation catheter is controlled by the radio frequency host to perform a radio frequency operation, the probe comes into contact with an operating site of the object of the radio frequency operation along with the central electrode, to detect the physical characteristic data of different positions in the operating site in real time. The physical characteristic data can be specifically temperature or impedance, or both temperature and impedance.

The object of the radio frequency operation refers to any object or object that can receive radio frequency operation such as radio frequency ablation. For example, when the radio frequency operation is radio frequency ablation, the object of the radio frequency operation may be a biological tissue, and the operating position may be an abnormal tissue in the biological tissue.

Step S302: obtaining a physical characteristic field of the object of the radio frequency operation according to the physical characteristic data acquired in real time.

Specifically, the physical characteristic data includes temperature data and impedance data, and correspondingly, the physical characteristic field can include a temperature field and a resistance field.

The temperature field is a set of temperatures at various points in the object of the radio frequency operation, reflecting the spatial and temporal distribution of temperature; which can generally be expressed as a function of spatial coordinates of an object and time. That is, t=f(x, y, z, τ), wherein x, y, and z are three rectangular coordinates in space, and τ is the time coordinate. In the prior art, many specific algorithms for temperature field are available, which are not particularly limited in the present application.

Similar to the temperature field, the impedance field is a set of impedances at various points in the object of the radio frequency operation, and is a function of time and spatial coordinates, reflecting the spatial and temporal distribution of impedance.

Step S303: obtaining the change of range of a to-be-operated area in a target operating area according to an initial range of the target operating area in the object of the radio frequency operation and the change of value of the physical characteristic data in the physical characteristic field, and displaying the change of range through a three-dimensional model.

The target operating area is an area to be implemented of the present radio frequency operation in the object of the radio frequency operation, and the initial range of the target operating area can be obtained by X-ray scanning and other transmission scanning techniques.

The to-be-operated area is an area where the present radio frequency operation has not been performed or the effect after the present radio frequency operation has not reached a standard, which still needs radio frequency operation.

The value of the physical characteristic data of each point in the physical characteristic field changes at any time as the radio frequency operation progresses, and the value of the physical characteristic data represents the stage of the radio frequency operation. Particularly, when the value of the physical characteristic data reaches a preset threshold, it indicates the end of the radio frequency operation (i.e., achieving the desired effect). When the value of the physical characteristic data is less than the preset threshold, it indicates that the radio frequency operation is not ended (i.e., not achieving the desired effect) or does not start. The area where the radio frequency operation is not ended or does not start is the to-be-operated area. That is, according to the value of the physical characteristic data in the physical characteristic field, the range of the to-be-operated area can be determined. As the measured value of each point in the physical characteristic field changes, the range of the to-be-operated area changes accordingly, showing such a trend that as the time of radio frequency operation increases, the range of the to-be-operated area becomes smaller and smaller.

By default 3D model display software, the change of range of the to-be-operated area in the target operating area is displayed on a display interface of the radio frequency host, to present it to a radio frequency operation personnel visually to get to know the status of the radio frequency operation.

In the embodiments of this application, multiple physical characteristic data of an operating position in an object of a radio frequency operation are acquired in real time by multiple probes, a physical characteristic field of the object of the radio frequency operation is obtained according to these data, then the change of range of a to-be-operated area in a target operating area is obtained according to an initial range of the target operating area in the object of the radio frequency operation and the change of value of the physical characteristic data in the physical characteristic field, and the range of change is displayed through a three-dimensional model. As a result, the visual prompting of the change of range of the to-be-operated area is achieved, the content of the prompt information is much rich, intuitive and vivid, and the accuracy and intelligence of determining the to-be-operated area is increased, thereby improving the effectiveness of information prompts, and thus improving the success rate and effect of the radio frequency operation.

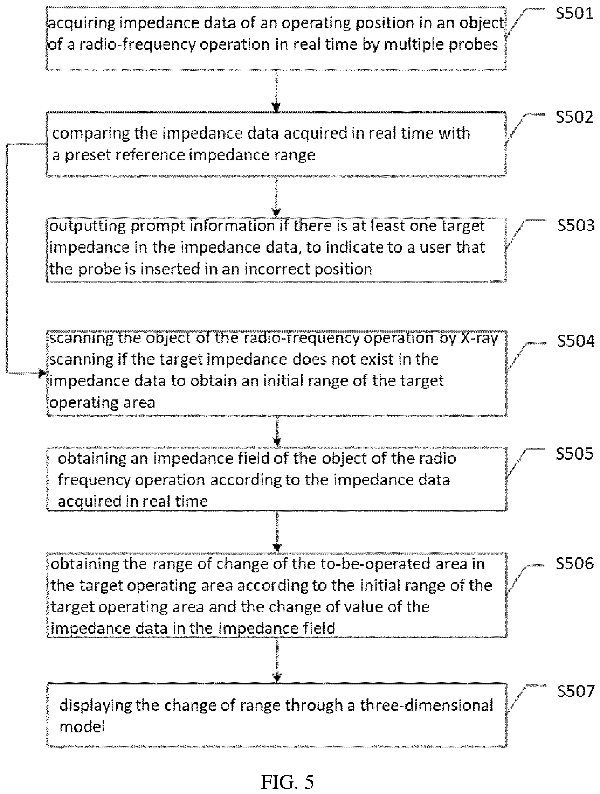

Fig. 5 shows a flow chart of a radio frequency operation prompting method provided in another embodiment of the present application. The method can be implemented by the radio frequency host 10 shown in FIG. 2, or implemented by other computer terminals connected thereto. For ease of description, in the following embodiments, the radio frequency host 10 is used as an implementation body. As shown in FIG. 5, the method specifically includes:

Step S501: acquiring impedance data of an operating position in an object of a radio frequency operation in real time by multiple probes.

Step S502: comparing the impedance data acquired in real time with a preset reference impedance range.

Step S503: outputting prompt information if there is at least one target impedance in the impedance data, to indicate to a user that the probe is inserted in an incorrect position.

As shown in FIG. 4, multiple probes 41 are arranged around a central electrode 42 provided at a tip of the radio frequency operation catheter 40, and located on different planes, to form a claw-shaped structure collectively. Each probe is provided with an impedance acquiring device, configured to acquire the impedance data of a pierced or touched position.

It can be understood that the impedance of normal biological tissues and the impedance of abnormal biological tissues are different, and a reference impedance database is configured in the radio frequency host 10. The reference impedance database is configured to store the reference impedance range corresponding to each of different types of abnormal biological tissues (e.g., tumors, inflammations, and cancers). By querying the reference impedance database, the reference impedance range corresponding to the type of abnormality in the object of the current radio frequency operation can be obtained. Optionally, the reference impedance database can also be configured in the cloud.

The value of the target impedance is not within the reference impedance range. The impedance data acquired by the multiple probes is compared with the reference impedance range queried, if there is at least one target impedance in the acquired impedance data, it means that the probe does not completely cover the site where the radio frequency operation needs to be performed, and the expected result may not be achieved if the radio frequency operation is performed at the current position. Consequently, preset prompt information is output on the display screen, to indicate to the user that the probe is inserted in an incorrect position.

Further, the prompt information also includes position information of a probe that obtains the target impedance, so that the user can determine a displacement direction of the probe according to the position information, making the information prompt more intelligent.

Further, after the prompt information is outputted, the process is returned to Step S501 every preset period of time until the target impedance does not exist in the impedance data; or in response to a control command triggered by the user by pressing a preset physical or virtual button, Step S501 is performed again.

Therefore, by using the reference impedance range, prompting is made when the probe is inserted in an incorrect position, to achieve the navigation of the positioning of the probe. This can make the information prompt more intelligent, and increase the speed of probe positioning, thus shortening the overall time of radio frequency operation, and improving the operation efficiency.

Step S504: scanning the object of the radio frequency operation by X-ray scanning if the target impedance does not exist in the impedance data to obtain an initial range of the target operating area.

Specifically, if the target impedance does not exist in the acquired impedance data, that is, all the acquired impedances fall into the reference impedance range, indicating that the probe completely covers the site where the radio frequency operation needs to be performed, a three-dimensional image of a target site is obtained by scanning the target site of the object of the radio frequency operation by X-ray scanning. Then the three-dimensional image is recognized, to obtain the position coordinates of each probe in the three-dimensional image, Then, the range of coverage of the probe is determined according to the obtained position coordinates, and the range of coverage is determined as the initial range of the target operating area. The X-ray scanning includes, for example, Computed Tomography (CT).

Step S505: obtaining an impedance field of the object of the radio frequency operation according to the impedance data acquired in real time.

Specifically, the impedance field is a set of impedances at various points in the object of the radio frequency operation, and is a function of time and spatial coordinates, reflecting the spatial and temporal distribution of impedance.

Step S506: obtaining the change of range of the to-be-operated area in the target operating area according to the initial range of the target operating area and the change of value of the impedance data in the impedance field.

The to-be-operated area is an area where the present radio frequency operation has not been performed or the effect after the present radio frequency operation has not reached the standard, which still needs radio frequency operation. The value of the impedance data of each point in the impedance field changes at any time as the radio frequency operation progresses, and the value of the impedance data represents the stage of the radio frequency operation. For example, when the value of the impedance data reaches a preset threshold, it indicates that the radio frequency operation achieves the desired effect. When the value of the impedance data is less than the preset threshold, it indicates that the radio frequency operation does not achieve the desired effect or does not start. The area where the radio frequency operation does not achieve the desired effect or does not start is the to-be-operated area. That is, according to the value of the impedance data in the impedance field, the range of the to-be-operated area can be determined. As the measured value of each point in the impedance field changes, the range of the to-be-operated area changes accordingly, showing such a trend that as the time of radio frequency operation increases, the range of the to-be-operated area becomes smaller and smaller.

Specifically, the value of the impedance data of each point in the impedance field is compared with a preset threshold (i.e., preset impedance threshold), and the boundary of the to-be-operated area is determined according to the points where the value of the impedance data is greater than the preset threshold.

It can be understood that the points where the value of the impedance data is greater than the preset threshold are fitted together by using a default fitting algorithm, to obtain the range of the area in the target operating area that has achieved the expected effect. The initial range of the target operating area is compared with the range of the area that has achieved the expected effect, to obtain the range and boundary of the to-be-operated area. The preset fitting algorithm includes, but is not limited to, for example, the least square method or the Matlab curve fitting algorithm, which is not particularly limited in the present application.

Further, when the value of the impedance data of the operating position is greater than the preset threshold, the range of a radiation area of the operating position is determined according to the value of the impedance data of the operating position, a detection angle of the probe corresponding to the operating position and a preset radiation distance; and the boundary of the to-be-operated area is determined according to the range of the radiation area.

It can be understood that since the radio frequency energy outputted by the central electrode of the radio frequency operation catheter is radiated into the biological tissue along a specific direction, the impedance change has a radiation range.

The detection angle of the probe, that is, the angle at which the probe used to detect the impedance of a certain operating position is pierced into or brought into contact with the operating position. According to the detection angle, the direction of radiation can be determined. According to the value of the impedance data of the operating position, the direction of the radiation, and the preset radiation distance, the range of the radiation area of the operating position, that is, the depth of the boundary of the range of the area that has achieved the desired effect, can be determined.

Step S507: displaying the change of range through a three-dimensional model.

Specifically, according to the three-dimensional image of the target operating area obtained by X-ray scanning and the change of range of the to-be-operated area in the target operating area, and by using algorithms such as Marching Cubes based on surface rendering, or Ray-casting, Shear-warp, Frequency Domain, and Splatting based on volume rendering, a three-dimensional model of change of range is established for the to-be-operated area, and displayed on a preset display interface.

In the Marching Cubes algorithm, a series of two-dimensional slice data is deemed as a three-dimensional data field, and then a three-dimensional surface mesh modeling a three-dimensional model is established by extracting the isosurface of the three-dimensional data. In this way, the three-dimensional model is established. The algorithm based on volume rendering is to directly convert the discrete data in a three-dimensional space into a final three-dimensional image, without generating intermediate geometric primitives. The central idea is to define an opacity for each voxel, take the transmission, emission and reflection of each voxel to light into account.

Optionally, in another embodiment of the present application, the multiple probes are configured to acquire temperature data of an operating position in an object of a radio frequency operation in real time, and the method includes the following steps:

Step S701: acquiring temperature data of an operating position in an object of a radio frequency operation in real time by multiple probes.

Step S702: obtaining an initial range of a target operating area by scanning the object of the radio frequency operation by X-ray scanning.

Step S703: obtaining a temperature field of the object of the radio frequency operation according to the temperature data acquired in real time.

Step S704: obtaining the range of change of a to-be-operated area in the target operating area according to the initial range of the target operating area and the change of value of the temperature data in the temperature field.

Step S705: displaying the change of range through a three-dimensional model.

The Steps S701 to S705 are similar to Step S501 and Steps S504 to S507 respectively, and related description can be made reference to Step S501 and Steps S504 to S507, and will not be repeated here.

Optionally, in another embodiment of the present application, the multiple probes are configured to acquire temperature data and impedance data of an operating position in an object of a radio frequency operation in real time, and the method includes the following steps:

Step S801: acquiring temperature data and impedance data of an operating position in an object of a radio frequency operation in real time by multiple probes.

Step S802: comparing the impedance data acquired in real time with a preset reference impedance range.

Step S803: outputting prompt information if there is at least one target impedance in the impedance data, to indicate to a user that the probe is inserted in an incorrect position.

Step S804: obtaining the initial range of the target operating area by scanning the object of the radio frequency operation by X-ray scanning, if the target impedance does not exist in the impedance data.

Step S805: obtaining an impedance field and a temperature field of the object of the radio frequency operation according to the impedance data and the temperature data acquired in real time.

Step S806: obtaining the range of change of a to-be-operated area in the target operating area according to the initial range of the target operating area, the change of value of the impedance data in the impedance field and the change of value of the temperature data in the temperature field.

Step S807: displaying the change of range through a three-dimensional model.

The Steps S801 to S805 and Step S807 are similar to Steps S501 to S505 and Step S507 respectively, and related description can be made reference to Step S501 and Steps S505 to S507, and will not be repeated here.

The difference is that the Step S805 specifically includes: comparing the value of the temperature data of each point in the temperature field with a preset temperature threshold, and determining a first boundary of the to-be-operated area according to points where the value of the temperature data is greater than the preset temperature threshold; and after a preset period of time, comparing the value of the impedance data at each point in the impedance field with a preset impedance threshold, and calibrating the first boundary of the to-be-operated area according to points where the value of the impedance data is greater than the preset impedance threshold, to obtain a second boundary, wherein the second boundary is determined as the boundary of the to-be-operated area.

The step of calibrating the first boundary of the to-be-operated area according to points where the value of the impedance data is greater than the preset impedance threshold to obtain a second boundary is that at the same point, if the value of the impedance data is greater than the preset impedance threshold, but the value of the temperature data is not greater than the preset temperature threshold, the impedance data is used as the basis, and the location corresponding to this point is determined as the location that achieves the desired effect.

Similarly, the first boundary is determined by using the temperature field, and then the first boundary is calibrated by using the impedance field, to achieve a complementary effect, and make the final boundary determined more accurate.

Optionally, the method also includes: determining the unit change of the to-be-operated area periodically according to the change of range of the to-be-operated area; and determining the remaining time before the end of the current radio frequency operation according to the unit change and the current volume of the to-be-operated area, and outputting the remaining time as prompt information on a display interface.

Specifically, an interval is determined according to a preset time, and the change of range of the to-be-operated area is divided by the past implementation time of the radio frequency operation every preset period of time, to obtain the unit change of the to-be-operated area, for example: the volume of the to-be-operated area reduced per second. Then, the current volume of the to-be-operated area is divided by the unit change, to obtain the remaining time before the end of the current radio frequency operation.

The initial volume of the target operating area minus the unit changes of the to-be-operated area is the current volume of the to-be-operated area. The initial volume of the target operating area can be determined based on the three-dimensional image of the target operating area obtained by X-ray scanning in Step S504.

Therefore, by prompting the remaining time before the end of the current radio frequency operation, it is possible to make the user know the process of radio frequency operation, thus further improving the intelligence of information prompts.

In the embodiments of this application, multiple pieces of physical characteristic data of an operating position in an object of a radio frequency operation are acquired in real time by multiple probes, a physical characteristic field of the object of the radio frequency operation is obtained according to these pieces of data, then the change of range of a to-be-operated area in a target operating area is obtained according to an initial range of the target operating area in the object of the radio frequency operation and the change of value of the physical characteristic data in the physical characteristic field, and the range of change is displayed through a three-dimensional model. As a result, the visual prompting of the change of range of the to-be-operated area is achieved, the content of the prompt information is much rich, intuitive and vivid, and the accuracy and intelligence of determining the to-be-operated area is increased, thereby improving the effectiveness of information prompts, and thus improving the success rate and effect of the radio frequency operation.

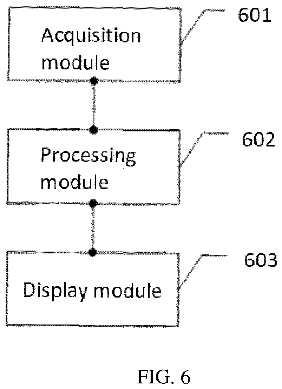

Fig. 6 is a schematic structural diagram of a radio frequency operation prompting device provided in an embodiment of the present application. For ease of description, only the parts relevant to the embodiments of the application are shown. The device may be a computer terminal, or a software module configured on the computer terminal. As shown in FIG. 6, the device includes an acquisition module 601, a processing module 602, and a display module 603.

The acquisition module 601 is configured to acquire physical characteristic data of an operating position in an object of a radio frequency operation in real time by multiple probes.

The processing module 602 is configured to obtain a physical characteristic field of the object of the radio frequency operation according to the physical characteristic data acquired in real time, and
obtain the change of range of a to-be-operated area in a target operating area according to an initial range of the target operating area in the object of the radio frequency operation and the change of value of the physical characteristic data in the physical characteristic field.

The display module 603 is configured to display the change of range through a three-dimensional model.

Further, the processing module 602 is further configured to compare the value of the physical characteristic data of each point in the physical characteristic field with a preset threshold; and determine a boundary of the range of the to-be-operated area according to points where the value of the physical characteristic data is greater than the preset threshold.

The processing module 602 is further configured to determine the range of a radiation area of the operating position according to the value of the physical characteristic data of the operating position, a detection angle of the probe corresponding to the operating position and a preset radiation distance, when the value of the physical characteristic data of the operating position is greater than the preset threshold. The value of the physical characteristic data in the radiation area is greater than the preset threshold; and the boundary of the to-be-operated area is determined according to the range of the radiation area.

Further, the physical characteristic data includes temperature data and/or impedance data, and the physical characteristic field includes a temperature field and/or a resistance field.

Further, when the physical characteristic data includes temperature data and impedance data, and the physical characteristic field includes a temperature field and a resistance field, the processing module 602 is further configured to compare the value of the temperature data of each point in the temperature field with a preset temperature threshold, and determine a first boundary of the to-be-operated area according to points where the value of the temperature data is greater than the preset temperature threshold; and
compare the value of the impedance data at each point in the impedance field with a preset impedance threshold after a preset period of time, and calibrate the first boundary of the to-be-operated area according to points where the value of the impedance data is greater than the preset impedance threshold to obtain a second boundary wherein the second boundary is determined as the boundary of the to-be-operated area.

Further, the processing module 602 is further configured to determine the unit change of the to-be-operated area periodically according to the change of range of the to-be-operated area; and determine the remaining time before the end of the current radio frequency operation according to the unit change and the current volume of the to-be-operated area.

The display module 603 is configured to output the remaining time as prompt information on a display interface.

Further, when the physical characteristic data includes impedance data, the processing module 602 is further configured to compare the impedance data acquired in real time with a preset reference impedance range, and trigger the display module 603 to output prompt information if there is at least one target impedance in the impedance data, to indicate to a user that the probe is inserted in an incorrect position, and the value of the target impedance is not within the reference impedance range;
and trigger the step of obtaining a physical characteristic field of the object of the radio frequency operation, according to the physical characteristic data acquired in real time, if the target impedance does not exist in the impedance data.

Further, the processing module 602 is further configured to scan the object of the radio frequency operation by X-ray scanning, to obtain the initial range of the target operating area.

The specific process for the above modules to implement their respective functions can be made reference to the relevant description in the embodiments shown in FIG. 3 to FIG. 5, and will not be repeated here.

In the embodiments of this application, multiple physical characteristic data of an operating position in an object of a radio frequency operation are acquired in real time by multiple probes, a physical characteristic field of the object of the radio frequency operation is obtained according to these data, then the change of range of a to-be-operated area in a target operating area is obtained according to an initial range of the target operating area in the object of the radio frequency operation and the change of value of the physical characteristic data in the physical characteristic field, and the range of change is displayed through a three-dimensional model. As a result, the visual prompting of the change of range of the to-be-operated area is realized, the content of the prompt information is much rich, intuitive and vivid, and the accuracy and intelligence of determining the to-be-operated area is increased, thereby improving the effectiveness of information prompts, and thus improving the success rate and effect of the radio frequency operation.

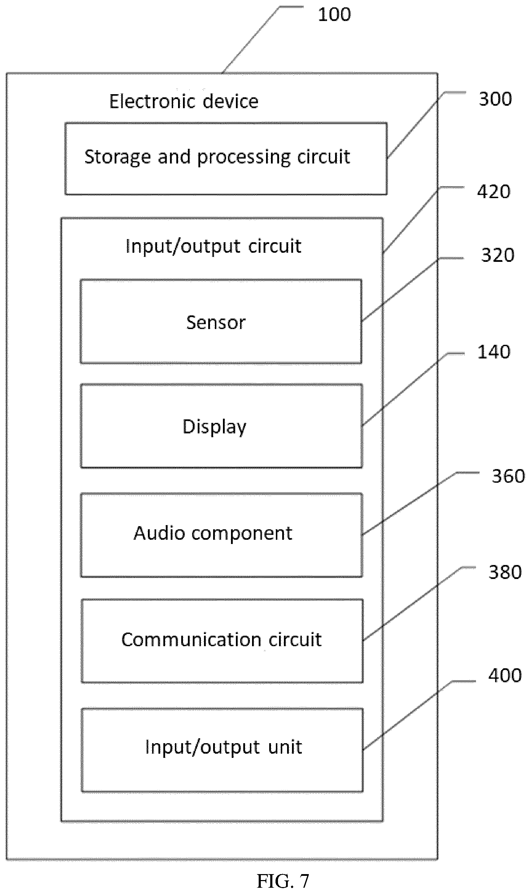

Fig. 7 is a schematic diagram showing a hardware structure of an electronic device provided in an embodiment of the present application.

Exemplarily, the electronic device may be any of various types of computer devices that are non-movable or movable or portable and capable of wireless or wired communication. Specifically, the electronic device can be a desktop computer, a server, a mobile phone or smart phone (e.g., a phone based on iPhone TM, and Android TM), a portable game device (e.g. Nintendo DS TM, PlayStation Portable TM, Gameboy Advance TM, iPhone TM), a laptop computer, PDA, a portable Internet device, a portable medical device, a smart camera, a music player, a data storage device, and other handheld devices such as watches, earphones, pendants, and headphones, etc. The electronic device may also be other wearable devices (for example, electronic glasses, electronic clothes, electronic bracelets, electronic necklaces and other head-mounted devices (HMD)).

As shown in FIG. 7, the electronic device 100 includes a control circuit, and the control circuit includes a storage and processing circuit 300. The storage and processing circuit 300 includes a storage, for example, hard drive storage, non-volatile storage (such as flash memory or other storages that are used to form solid-state drives and are electronically programmable to confine the deletion, etc.), and volatile storage (such as static or dynamic random access storage) which is not limited in the embodiments of the present application. The processing circuit in the storage and processing circuit 300 can be used to control the operation of the electronic device 100. The processing circuit can be implemented based on one or more microprocessors, microcontrollers, digital signal processors, baseband processors, power management units, audio codec chips, application specific integrated circuits, and display driver integrated circuits.

The storage and processing circuit 300 can be used to run the software in the electronic device 100, such as Internet browsing applications, Voice over Internet Protocol (VOIP) phone call applications, Email applications, media player applications, and functions of operating system. The software can be used to perform some control operations, For example, camera-based image acquisition, ambient light measurement based on an ambient light sensor, proximity sensor measurement based on a proximity sensor, information display function implemented by a status indicator based on a status indicator lamp such as light emitting diode, touch event detection based on a touch sensor, functions associated with displaying information on multiple (e.g. layered) displays, operations associated with performing wireless communication functions, operations associated with the collection and generation of audio signals, control operations associated with the collection and processing of button press event data, and other functions in electronic device 100, which is not limited in the embodiments of the present application.

Further, the storage stores an executable program code. The processor coupled to the storage calls the executable program code stored in the storage, and implements the radio frequency operation prompting method described in various embodiments shown above.

The executable program code includes various modules in the radio frequency operation prompting device described in the embodiment shown in FIG. 6, for example, the acquisition module 601, the processing module 602, and the display module 603. The specific process for the above modules to implement their functions can be made reference to the relevant description in the embodiments shown in FIG. 6, and will not be repeated here.

The electronic device 100 may also include an input/output circuit 420. The input/output circuit 420 can be used to enable the electronic device 100 to implement the input and output of data, that is, the electronic device 100 is allowed to receive data from an external device and the electronic device 100 is also allowed to output data from the electronic device 100 to the external device. The input/output circuit 420 may further include a sensor 320. The sensor 320 may include one or a combination of an ambient light sensor, a proximity sensor based on light and capacitance, a touch sensor (e.g., light-based touch sensor and/or capacitive touch sensor, wherein, the touch sensor may be part of the touch screen, or it can also be used independently as a touch sensor structure), an accelerometer, and other sensors, etc.

The input/output circuit 420 may also include one or more displays, for example, display 140. The display 140 may include a liquid crystal display, an organic light emitting diode display, an electronic ink display, a plasma display, and a display that uses other display technologies. The display 140 may include a touch sensor array (i.e., the display 140 may be a touch display screen). The touch sensor can be a capacitive touch sensor formed by an array of transparent touch sensor electrodes (such as indium tin oxide (ITO) electrodes), or a touch sensor formed using other touch technologies, for example, sonic touch, pressure sensitive touch, resistive touch, and optical touch, which is not limited in the embodiments of the present application.

The electronic device 100 can also include an audio component 360. The audio component 360 can be used to provide audio input and output functions for the electronic device 100. The audio component 360 in the electronic device 100 includes a speaker, a microphone, a buzzer, and a tone generator and other components used to generate and detect sound.

A communication circuit 380 can be used to provide the electronic device 100 with an ability to communicate with an external device. The communication circuit 380 may include an analog and digital input/output interface circuit, and a wireless communication circuit based on radio frequency signals and/or optical signals. The wireless communication circuit in the communication circuit 380 may include a radio frequency transceiver circuit, a power amplifier circuit, a low-noise amplifier, a switch, a filter, and an antenna. For example, the wireless communication circuit in the communication circuit 380 may include a circuit for supporting near field communication (NFC) by transmitting and receiving near-field coupled electromagnetic signals. For example, the communication circuit 380 may include a near-field communication antenna and a near-field communication transceiver. The communication circuit 380 may also include a cellular phone transceiver and antenna, and a wireless LAN transceiver circuit and antenna, etc.

The electronic device 100 may also further include a battery, a power management circuit and other input/output units 400. The input/output unit 400 may include a button, a joystick, a click wheel, a scroll wheel, a touchpad, a keypad, a keyboard, a camera, a light-emitting diode and other status indicators, etc.

The user can control the operation of the electronic device 100 by inputting a command through the input/output circuit 420, and the output data from the input/output circuit 420 enables the receiving of status information and other outputs from the electronic device 100.

Further, an embodiment of the present application further provides a non-transitory computer-readable storage medium. The non-transitory computer-readable storage medium can be configured in the server in each of the above embodiments, and a computer program is stored in the non-transitory computer-readable storage medium. When the program is executed by the processor, the radio frequency operation prompting method described in the embodiments is implemented.

In the embodiments described above, emphasis has been placed on the description of various embodiments. Parts of an embodiment that are not described or illustrated in detail may be found in the description of other embodiments.

It can be recognized by those skilled in the art that the exemplary modules/units and algorithm steps described in connection with embodiments disclosed herein can be implemented by electronic hardware, or a combination of computer software and electronic hardware. Whether these functions are implemented by hardware or software depends on the specific constraints for application and design of the technical solution. For each specific application, different methods can be used by professional and technical personnel to implement the described functions. However, this implementation should not be considered as going beyond the scope of the present invention.

In the embodiments provided in the present invention, it can be understood that the disclosed device/terminal and method can be implemented in other ways. For example, the device/terminal embodiments described above are merely illustrative. For example, a division of the modules or elements is merely division of logical functions, and there may be additional divisions in actual implementation. For example, multiple units or components may be combined or integrated into another system, or some features may be omitted or not performed. Alternatively, the couplings or direct couplings or communicative connections shown or discussed with respect to one another may be indirect couplings or communicative connections via some interfaces, devices or units may be electrical, mechanical or otherwise.

The units described as separate components may or may not be physically separate, and the components shown as units may or may not be physical units, i.e. may be located in one place, or may be distributed over a plurality of network elements. Some or all of the units may be selected to achieve the objectives of the solution of the present embodiment according to practical requirements.

In addition, the functional units in the various embodiments of the present invention may be integrated into one processing unit, may be physically separate from each other or may be integrated in one unit by two or more units. The integrated units described above can be implemented either in the form of hardware, or software functional units.

The integrated unit, if implemented in the form of a software functional unit and sold or used as a stand-alone product, may be stored in a computer readable storage medium. Based on such an understanding, all or part of the processes of the methods in the above-described embodiments implemented in the present application, may also be implemented by a computer program instructing related hardware. The computer program may be stored in a computer-readable storage medium, and performs the steps of the various method embodiments described above when executed by the processor. The computer program includes a computer program code, which may be in the form of source code, object code, or executable file, or in some intermediate form. The computer readable medium may include: any entity or device capable of carrying the computer program code, recording media, U disks, removable hard disks, magnetic disks, optical disks, computer memory, read-only memory (ROM), random access memory (RAM), electric carrier wave signals, telecommunication signals and software distribution media. It should be noted that the computer readable medium may contain content that may be appropriately augmented or subtracted as required by legislation and patent practice within judicial jurisdictions, e.g., the computer-readable medium does not include electrical carrier wave signals and telecommunications signals in accordance with legislation and patent practices in some jurisdictions.

The above-described embodiments are merely illustrative of, and not intended to limit the technical solutions of the present invention. Although the present invention has been described in detail with reference to the foregoing embodiments, it should be understood by those of skill in the art that modifications can be made to technical solutions described in the foregoing embodiments, or some of the technical features thereof can be equivalently substituted; and such modifications and substitutions do not cause the nature of the corresponding technical solution to depart from the spirit and scope of the embodiments of the present disclosure and are intended to be included within the scope of this application.

## Claims

1. A radio frequency operation prompting method, applicable to a computer terminal, comprising steps of:
acquiring physical characteristic data of an operating position in an object of a radio frequency operation in real time by multiple probes;
obtaining a physical characteristic field of the object of the radio frequency operation according to the physical characteristic data acquired in real time, and
obtaining a change of range of a to-be-operated area in a target operating area according to an initial range of the target operating area in the object of the radio frequency operation and a change of value of the physical characteristic data in the physical characteristic field, and displaying the change of range through a three-dimensional model.

2. The method according to claim 1, wherein the step of obtaining a change of range of a to-be-operated area in a target operating area according to an initial range of the target operating area in the object of the radio frequency operation and a change of value of the physical characteristic data in the physical characteristic field comprises steps of:
comparing the value of the physical characteristic data of each point in the physical characteristic field with a preset threshold; and
determining a boundary of the to-be-operated area according to points where the value of the physical characteristic data is greater than the preset threshold.

3. The method according to claim 1 or 2, wherein the step of obtaining a change of range of a to-be-operated area in a target operating area according to an initial range of the target operating area in the object of the radio frequency operation and a change of value of the physical characteristic data in the physical characteristic field further comprises steps of:
determining a range of a radiation area of the operating position according to the value of the physical characteristic data of the operating position, a detection angle of the probe corresponding to the operating position and a preset radiation distance when the value of the physical characteristic data of the operating position is greater than the preset threshold, wherein the value of the physical characteristic data in the radiation area is greater than the preset threshold; and
determining a boundary of the to-be-operated area according to the range of the radiation area.

4. The method according to claim 3, wherein the physical characteristic data comprises temperature data and/or impedance data, and the physical characteristic field comprises a temperature field and/or a resistance field.

5. The method according to claim 4, wherein when the physical characteristic data comprises temperature data and impedance data, and the physical characteristic field comprises a temperature field and a resistance field, the step of obtaining a change of range of a to-be-operated area in a target operating area according to an initial range of the target operating area in the object of the radio frequency operation and a change of value of the physical characteristic data in the physical characteristic field comprises steps of:
comparing a value of the temperature data of each point in the temperature field with a preset temperature threshold, and determining a first boundary of the to-be-operated area according to points where the value of the temperature data is greater than the preset temperature threshold; and
comparing a value of the impedance data at each point in the impedance field with a preset impedance threshold after a preset period of time, and calibrating the first boundary of the to-be-operated area according to points where the value of the impedance data is greater than the preset impedance threshold to obtain a second boundary, wherein the second boundary is determined as the boundary of the to-be-operated area.

6. The method according to claim 5, further comprising steps of:
determining unit change of the to-be-operated area periodically according to the change of range of the to-be-operated area; and
determining the remaining time before end of the current radio frequency operation according to the unit change and current volume of the to-be-operated area, and outputting the remaining time as prompt information on a display interface.

7. The method according to claim 1, wherein when the physical characteristic data comprises impedance data, before the step of obtaining a physical characteristic field of the object of the radio frequency operation according to the physical characteristic data acquired in real time, the method further comprises steps of:
comparing the impedance data acquired in real time with a preset reference impedance range, and
outputting prompt information when there is at least one target impedance in the impedance data, to indicate to a user that the probe is inserted in an incorrect position, and a value of the target impedance is not within the reference impedance range; and implementing the step of obtaining a physical characteristic field of the object of the radio frequency operation, according to the physical characteristic data acquired in real time when the target impedance does not exist in the impedance data.

8. The method according to any one of claims 1 to 6, further comprising: before the step of obtaining a physical characteristic field of the object of the radio frequency operation according to the physical characteristic data acquired in real time, a step of: scanning the object of the radio frequency operation by X-ray scanning, to obtain the initial range of the target operating area.

9. A radio frequency operation prompting device, comprising:
an acquisition module, configured to acquire physical characteristic data of an operating position in an object of a radio frequency operation in real time by multiple probes;
a processing module, configured to obtain a physical characteristic field of the object of the radio frequency operation according to the physical characteristic data acquired in real time, and
obtain a change of range of a to-be-operated area in a target operating area according to an initial range of the target operating area in the object of the radio frequency operation and a change of value of the physical characteristic data in the physical characteristic field; and
a display module, configured to display the change of range through a three-dimensional model.

10. An electronic device, comprising:
a storage and a processor, wherein
the storage stores an executable program code; and
the processor is coupled to the storage, and configured to call the executable program code stored in the storage, and implement each step of the radio frequency operation prompting method according to any one of claims 1 to 8.

11. A non-transitory computer-readable storage medium, on which a computer program is stored, wherein when the computer program is executed by a processor, the radio frequency operation prompting method according to any one of claims 1 to 8 is implemented.
